# FASCICULE DE BREVET EUROPEEN

(11) **EP 3 448 527 B1**
(45) Date de publication et mention de la délivrance du brevet: **26.08.2020**
(21) Numéro de dépôt: 17713292.5
(22) Date de dépôt: 24.03.2017
(51) Int. Cl.: A61Q 15/00, G01N 15/08, B01L 3/00, G01N 33/487

(54) **SYSTEME D'EVALUATION IN VITRO DE L'EFFICACITE D'UN DEODORANT**
IN-VITRO-SYSTEM ZUR BEURTEILUNG DER WIRKSAMKEIT EINES DEODORANTS
IN VITRO SYSTEM FOR EVALUATING THE EFFECTIVENESS OF A DEODORANT

(30) Priorité: 27.04.2016 FR 1653752
(43) Date de publication de la demande: 06.03.2019
(73) Titulaire: Microfactory, 75005 Paris (FR)
(72) Inventeur: DUPIRE, Jules, 75013 Paris (FR); MONTI, Fabrice, 91160 Saulx les Chartreux (FR); TABELING, Patrick, 75013 Paris (FR)
(74) Mandataire: Regimbeau
(86) Numéro de dépôt international: PCT/EP2017/057050
(87) Numéro de publication internationale: WO 2017/186423

(56) Documents cités:
- WO-A1-2014/170174
- US-A- 4 884 438
- LINLIN HOU ET AL: "Artificial microfluidic skin for in vitro perspiration simulation and testing", LAB ON A CHIP: MINIATURISATION FOR CHEMISTRY, PHYSICS, BIOLOGY, MATERIALS SCIENCE AND BIOENGINEERING, vol. 13, no. 10, 18 mars 2013 (2013-03-18) , page 1868, XP055328483, GB ISSN: 1473-0197, DOI: 10.1039/c3lc41231h

## Description

La présente invention s'applique aux domaines de la cosmétique, de la pharmacie et de la médecine. Elle concerne un système d'évaluation de l'efficacité d'un déodorant et plus précisément un système permettant de modéliser *in vitro* l'interaction entre la sueur et un déodorant.

Les déodorants permettent de limiter les odeurs corporelles en empêchant la décomposition bactérienne de la transpiration. Les anti-transpirants, un sous-groupe des déodorants, permettent de réguler et/ou d'arrêter l'émission de sueur sur la peau. Cette diminution ou cet arrêt de la transpiration peut être réalisé en choisissant un déodorant comportant notamment des sels d'aluminium, comme l'hydrochlorure d'aluminium et/ou des sels de zirconium. Pour bloquer l'émission de sueur, un déodorant peut entraîner la formation d'un bouchon dans un pore sudoral. Un des mécanismes proposés pour expliquer la formation de ce bouchon implique une étape formation de flocs dans un pore sudoral, c'est-à-dire dire une agglomération de protéines de la sueur en particules aptes à boucher un pore. D'autres mécanismes ont été proposés pour expliquer la formation de ces bouchons.

L'Agence Française de Sécurité Sanitaire des Produits de Santé (AFSAPS) rapporte une toxicité chez certains patients d'une exposition répétée à l'aluminium et préconise l'utilisation de produits anti-transpirants dont la concentration massique en sel d'aluminium est inférieure à 2%, alors qu'elle est généralement comprise entre 5% et 20% dans les produits sur le marché, selon les modes d'application considérés (Évaluation du risque lié à l'utilisation de l'aluminium dans les produits cosmétiques, AFSAPS, 2011).

Ce risque sanitaire entraîne un objectif technique général consistant à produire un déodorant de type anti-transpirant efficace qui comporte des concentrations en hydrochlorure d'aluminium inférieures à celles du commerce, alors que les déodorants sont moins efficaces pour de faibles concentrations d'hydrochlorure d'aluminium. Ce problème peut être résolu de différentes manières. On peut par exemple utiliser d'autres produits déodorants. WO 2010017609 divulgue par exemple des produits anti-transpirants comprenant un agoniste des canaux ioniques TRMP8 de manière à réduire la production de sueur. Il est également possible de pister les molécules les plus aptes à être des anti-transpirants parmi une large gamme. WO 2015072970 divulgue par exemple une mesure du potentiel ζ pour évaluer l'efficacité d'un produit anti-transpirant, ce potentiel étant corrélé à la cinétique de floculation de protéines de la sueur au contact d'un produit anti-transpirant.

LINLIN HOU ET AL: "Artificial microfluidic skin for in vitro perspiration simulation and testing",LAB ON A CHIP: MINIATURISATION FOR CHEMISTRY, PHYSICS, BIOLOGY, MATERIALS SCIENCE AND BIOENGINEERING, vol. 13, no. 10, 18 mars 2013 (2013-03-18), page 1868, XP055328483, ISSN: 1473-0197, DOI: 10.1039/c3lc41231h, divulgue une peau artificielle comprenant un dispositif fluidique avec un réservoir et un canal de liaison, permettant une circulation de sueur artificielle.

WO 2014170174 divulgue un système micro fluidique, dans lequel des canaux aptes à transporter de la sueur sont reliés à un canal adapté à l'écoulement d'un produit comprenant un produit anti-transpirant. L'interaction de la sueur et du produit anti-transpirant sous forme liquide entraîne la formation d'un bouchon qui peut être observé par imagerie. D'une part, cette méthode ne permet pas d'évaluer les formes galéniques les plus courantes des produits anti-transpirants. D'autre part, les pores sudoraux sont modélisés par des canaux microfluidiques dont la largeur peut être de l'ordre de la dizaine de micromètres. En pratique, il est problématique de réussir à déplacer un ménisque (interface fluide-air), formé par l'injection de sueur dans un canal, jusqu'au point de jonction exact avec un canal apte à transporter un produit transpirant sous forme liquide, ainsi que de le stabiliser à ce point. En effet, une faible variation du volume de sueur injectée, par exemple de l'ordre de la dizaine de picolitre, peut entraîner un déplacement du ménisque d'une centaine de micromètres.

Pour amener le ménisque et le stabiliser à un point de jonction, une première solution consiste à injecter de la sueur dans un canal, l'injection étant réalisée par une source de débit, par exemple un pousse-seringue, puis à couper la connexion fluidique entre la source de débit et le canal, par exemple à l'aide d'un robinet. Dans ce cas, aucun contrôle n'est possible sur la sueur injectée dans le canal une fois la connexion coupée, en particulier si la connexion fluidique et le canal sont réalisés en matériau rigide. Le ménisque peut se déplacer librement et il n'est pas possible de contrôler le lieu de la réaction avec un produit anti-transpirant.

Une seconde solution consiste à injecter de la sueur dans un canal avec une source de débit, par exemple un pousse-seringue, puis à imposer un débit nul en commandant la source de débit. Les pousse-seringue peuvent par exemple être caractérisés par l'actuation de très faibles débits, par exemple de l'ordre de 1 pL/s. Toutefois, le temps de stabilisation d'un pousse-seringue est trop important (par exemple de l'ordre de quelques minutes) pour pouvoir stabiliser la position du ménisque à la jonction entre deux canaux.

Une troisième solution consiste à injecter de la sueur dans un canal en utilisant un actuateur de pression (ou contrôleur de pression). Un actuateur de pression peut imposer une pression, par exemple supérieure à la pression atmosphérique, à la phase gazeuse d'un réservoir hermétique au gaz, dans lequel est agencée une source de sueur liquide. Cette pression imposée permet d'injecter la sueur dans un canal relié à la sueur du réservoir. Les actuateurs de pression sont caractérisés par un temps de réponse plus court que celui des sources de débit. Toutefois, un actuateur de pression ne permet d'appliquer des pressions entraînant un écoulement que dans un seul sens. Il n'est pas possible d'imposer à la fois des pressions positives et négatives avec le même actuateur, sans transiger avec un temps de réponse plus long.

De plus, la relaxation mécanique (par exemple élastique) du matériau formant les canaux fluidiques et/ou des connectiques fluidiques (par exemple due à une variation de pression ou de température) peut entraîner une dérive du débit malgré l'imposition d'un débit nul par une source de débit ou un actuateur de pression. Enfin, des phénomènes de capillarité peuvent créer des variations de pression à l'échelle microscopique au voisinage du ménisque, entraînant un mouvement du ménisque et empêchant sa stabilisation. De fait, les conditions expérimentales de l'étude de formation des bouchons sont peu reproductibles, sensibles et difficiles à stabiliser.

L'instabilité d'un ménisque à un point de jonction empêche également de réaliser des évaluations *in vitro* d'un déodorant d'un autre type qu'un anti-transpirant. Elle présente un problème technique pour l'étude de produits antibactériens ou de produits hybrides comportant des antibactériens et des anti-transpirants.

L'invention vise à remédier à une partie ou à la totalité des inconvénients précités de l'art antérieur, et plus particulièrement à réaliser un système apte à maintenir un ménisque de sueur à l'extrémité d'un canal apte à transporter la sueur, à débit nul et stable sans limite dans le temps comparativement au temps d'une expérience d'évaluation de l'efficacité d'un ou de plusieurs produits déodorants.

Un objet de l'invention permettant d'atteindre ce but, partiellement ou totalement, est un système d'évaluation *in vitro* de l'efficacité d'un produit déodorant comprenant :
- au moins un dispositif fluidique comprenant au moins un canal principal comportant une entrée à l'une de ses extrémités et apte à la circulation de sueur naturelle ou artificielle ;
- au moins un réservoir apte à contenir de la sueur naturelle ou artificielle et une phase gazeuse, séparés par une interface à une hauteur h1,
- au moins un canal de liaison relié à un dit réservoir et à l'entrée d'au moins un dit canal principal ;
- un actuateur de pression apte à imposer la pression d'une phase gazeuse à l'intérieur dudit réservoir ;
caractérisé en ce qu'il comporte également :
- au moins un débitmètre ;
- au moins un régulateur par asservissement en boucle fermée ;
un dit débitmètre étant apte à mesurer le débit d'un dit canal principal et à transmettre une information dudit débit audit régulateur, ledit régulateur étant apte à transmettre une information de régulation au dit actuateur de pression, ledit canal principal présentant un orifice formé par sa paroi à son autre extrémité, à une hauteur h₂, ladite hauteur h₁ étant strictement différente de h₂.

Avantageusement, h₂ et strictement supérieure à h₁ et un dit actuateur de pression du système est apte à imposer une pression supérieure à la pression atmosphérique dans un dit réservoir.

Avantageusement, ledit dispositif fluidique du système comporte une pluralité de canaux principaux, lesdits canaux principaux étant indépendants.

Avantageusement, la largeur maximale d'un dit orifice du système est inférieure ou égale à 1 mm.

Avantageusement, un dit dispositif fluidique du système est agencé dans une enceinte dans laquelle au moins un élément choisi parmi l'hygrométrie et la température est commandé.

Avantageusement, un dispositif fluidique du système comprend une première partie monolithique comprenant chaque dit canal principal et au moins une face extérieure sensiblement plane, chaque orifice coïncidant avec la face extérieure.

Avantageusement, le dispositif fluidique comporte au moins un capteur choisi parmi un capteur de pH, un capteur d'acides aminés, un capteur de nucléotides, un capteur enzymatique et un capteur de bactéries, chaque dit capteur étant agencé sur une surface choisie parmi une paroi d'un dit canal principal et une dite face extérieure.

Un autre objet de l'invention est un procédé d'évaluation de l'efficacité d'un produit déodorant mis en œuvre par un système comprenant au moins un dispositif fluidique comprenant au moins un canal principal, chaque dit canal principal comprenant une entrée à l'une de ses extrémités et présentant un orifice formé par la paroi dudit canal principal à une autre de ses extrémités à une hauteur h₂, ledit système comprenant au moins un réservoir apte à contenir de la sueur naturelle ou artificielle et une phase gazeuse, séparés par une interface à une hauteur h₁, h₁ étant strictement différente de h₂, ledit procédé comprenant au moins les étapes consistant à :
- introduire de la sueur naturelle ou artificielle dans l'ensemble d'un dit canal principal en imposant une pression dans la phase gazeuse d'un réservoir comportant de ladite sueur, avec un actuateur de pression, ledit réservoir étant fluidiquement relié audit canal principal ;
- mesurer un débit de sueur dudit canal principal, transmettre l'information dudit débit à un régulateur, ledit régulateur imposant une consigne de pression audit actuateur de pression, de manière à imposer un débit sensiblement nul audit canal principal par asservissement en boucle fermée ;
- mettre en contact ladite sueur et un produit déodorant de part et d'autre d'au moins un dit orifice ;
- attendre pendant le déroulement d'une réaction entre ladite sueur et ledit produit déodorant ;
- déterminer au moins un paramètre choisi parmi un paramètre physico-chimique et un paramètre biologique caractéristique d'une dite réaction entre ladite sueur et un dit produit déodorant.

Avantageusement, ledit produit déodorant est sous une forme galénique choisie parmi au moins un gel, une crème, une émulsion, une mousse et une solution anhydre.

Avantageusement, le procédé comprend une étape de consistant à absorber la sueur en contact avec un dit orifice à l'extérieur d'un dit canal principal, entre la seconde étape et la troisième étape d'un procédé selon la revendication.

Avantageusement, la quatrième étape du procédé consiste au moins à augmenter la pression d'une dite phase gazeuse, mesurer le débit d'un dit canal principale et à mesurer la pression de la dite phase gazeuse lors d'une variation sensible du débit dudit canal principal.

Avantageusement, on observe la formation d'un dit bouchon par imagerie pendant la quatrième étape du procédé.

Avantageusement, la deuxième étape du procédé est réalisée en pulvérisant un dit produit déodorant sur un dit orifice.

Avantageusement, le procédé met en œuvre un dit dispositif fluidique comprenant une première partie monolithique, ladite première partie comprenant chaque dit canal principal et au moins une face extérieure sensiblement plane, chaque dit orifice coïncidant avec ladite face extérieure et l'étape de mise en contact ladite sueur et un produit déodorant de part et d'autre d'au moins un dit orifice est réalisée en déposant un dit produit déodorant sur ladite face extérieure sensiblement plane.

Avantageusement, la cinquième étape du procédé comprend une mesure du pH d'au moins un élément choisi parmi une dite sueur, un dit produit déodorant et un produit de ladite réaction entre une dite sueur et un dit produit déodorant.

Avantageusement, ledit dispositif fluidique est sensiblement transparent et la cinquième étape du procédé comprend une mesure d'un élément choisi parmi la transmission et réflexion d'un éclairage lumineux émis vers au moins un élément choisi parmi une dite sueur, un dit produit déodorant et un produit de ladite réaction entre une dite sueur et un dit produit déodorant.

Avantageusement, la cinquième étape du procédé comprend une détection de la présence de bactéries dans au moins un élément choisi parmi une dite sueur, un dit produit déodorant et un produit de ladite réaction entre une dite sueur et un dit produit déodorant.

Avantageusement, la troisième étape du procédé est réalisée en déposant une cale d'épaisseur e₁ sur ladite face extérieure, ladite cale étant ajourée au dessus d'au moins un dit orifice, et en raclant un produit déodorant sur ladite cale de manière à déposer une couche de produit déodorant sensiblement d'épaisseur e₁ au dessus d'au moins un dit orifice.

L'invention sera mieux comprise et d'autres avantages, détails et caractéristiques de celle-ci apparaîtront au cours de la description explicative qui suit, faite à titre d'exemple en référence aux dessins annexés dans lesquels :
- la figure 1 illustre schématiquement un système 1 d'évaluation *in vitro* de l'efficacité d'un produit déodorant 2 selon un mode de réalisation de l'invention ;
- la figure 2 illustre schématiquement un dispositif fluidique 3 ;
- la figure 3 est une photographie réalisée par un microscope électronique à balayage illustrant des orifices 11 d'un dispositif fluidique 3 ;
- la figure 4 est une photographie réalisée par un microscope électronique à balayage illustrant un orifice 11 d'un dispositif fluidique 3 ;
- la figure 5 est une photographie illustrant un dispositif fluidique 3 et des canaux de liaison 10 ;
- la figure 6 est un schéma illustrant un procédé selon un mode de réalisation de l'invention ;
- la figure 7 est une photographie microscopique illustrant la formation de bouchons 25 dans un dispositif fluidique 3 ;
- la figure 8 est un diagramme illustrant une mesure du débit dans un canal principal 4 en fonction du temps lors de la mise en œuvre d'un procédé selon un mode de réalisation de l'invention ;
- la figure 9 est un diagramme illustrant une mesure de la pression de débouchage et
- la figure 10 illustre une méthode de dépôt et de mise en contact d'un produit déodorant 2 avec la sueur 5.

On entend par sueur 5 naturelle un liquide biologique sécrété par les glandes sudorales lors de la transpiration. La sueur 5 naturelle a par exemple été préalablement prélevée à partir d'un être humain par toute méthode connue de l'homme du métier.

On entend par sueur 5 artificielle une sueur 5 comprenant par exemple des éléments dont du chlorure de sodium (NaCl), de l'acide lactique, de l'urée, de la BSA (acronyme de *bovine serum albumin* en anglais soit albumine de sérum bovin), de l'ammoniaque en quantité suffisante pour obtenir un pH sensiblement neutre et de l'eau. En variante, tout autre type de sueur artificielle connu de l'homme du métier peut être utilisé dans le cadre de l'invention.

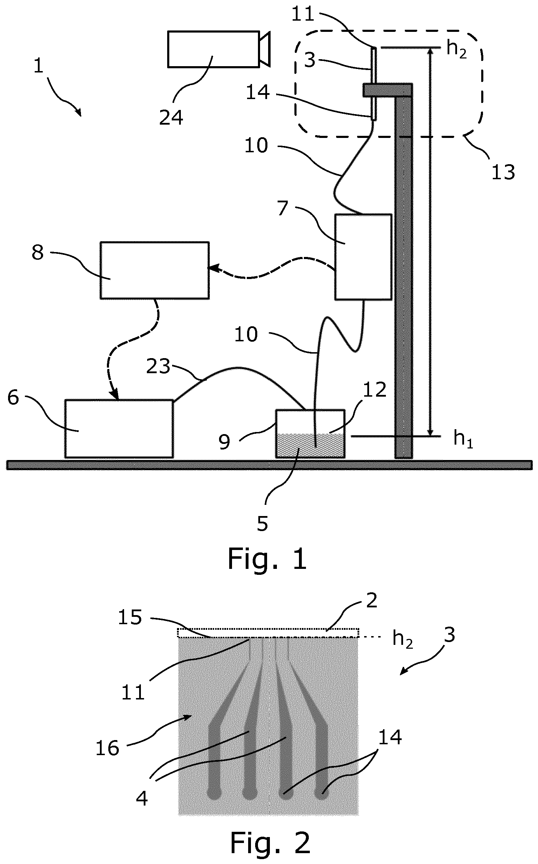

La figure 1 illustre schématiquement un système 1 d'évaluation *in vitro* de l'efficacité d'un produit déodorant 2, selon un mode de réalisation de l'invention.

Le système 1 comprend un actuateur de pression 6, aussi appelé contrôleur de pression, apte à imposer une pression à une ou plusieurs sorties. L'actuateur de pression 6 peut comporter une pluralité de sorties dans lesquelles les pressions imposées sont indépendantes et/ou couplées. Une sortie de l'actuateur de pression 6 est reliée à un tuyau d'imposition de la pression 23, lui-même relié à un réservoir 9. De cette manière, l'actuateur de pression 6 permet d'imposer la pression d'une phase gazeuse d'un réservoir 9.

Le système 1 comprend également au moins un réservoir 9 apte à contenir de la sueur 5 naturelle ou artificielle et une phase gazeuse. La phase liquide, c'est-à-dire la sueur 5, et la phase gazeuse, typiquement de l'air, sont séparées par une interface 12 à une hauteur h₁. Typiquement, un réservoir peut contenir entre 0,1 mL et 100 mL de sueur 5.

Un tuyau d'imposition de la pression 23 est relié à la phase gazeuse d'un réservoir 9 par une ouverture du réservoir 9. Ainsi, l'actuateur de pression 6 peut imposer une pression dans la phase gazeuse d'un réservoir 9. Si la pression imposée est supérieure à la pression atmosphérique, la surpression correspondante peut être comprise entre 0 bar et 20 bar et préférentiellement comprise entre 0 bar et 2 bar. Typiquement, une variation de pression peut être imposée dans la phase gazeuse par l'actuateur de pression 6 en moins de 200 ms, préférentiellement en moins de 100 ms et préférentiellement en moins de 50 ms.

Un canal de liaison 10 est relié à un réservoir 9 par une ouverture du réservoir 9 permettant d'agencer une extrémité du canal de liaison 10 dans la sueur 5. Le réservoir reste hermétique à toute phase gazeuse de l'environnement extérieur dans cet agencement. En variante, on peut relier un canal de liaison 10 à une ouverture d'un réservoir 9 en contact avec la sueur 5. Le canal de liaison 10 relie le réservoir 9 à l'entrée 14 d'un canal principal 4.

Par canal de liaison 10, on entend un canal, un tuyau ou une tubulure, par exemple souple, permettant de relier le réservoir 9 à l'entrée 14. On entend également par canal de liaison 10 toute succession de canaux reliés en série permettant de relier le réservoir 9 à l'entrée 14, par exemple une tubulure reliée à un manchon métallique, ou une tubulure reliée au canal d'un instrument de mesure lui-même relié à une autre tubulure.

Le système 1 comprend également un dispositif fluidique 3. Un dispositif fluidique 3 comprend au moins un canal principal 4, apte à la circulation de sueur 5. Le canal principal 4 comprend une entrée 14 à l'une de ses extrémités et présente un orifice 11, formé par sa paroi à son autre extrémité. Cet orifice 11 peut être vu comme une modélisation de l'extrémité d'un canal sudoral en contact avec l'environnement extérieur. Dans l'ensemble des modes de réalisation de l'invention, un orifice 11 est agencé à une hauteur h₂, strictement différente de la hauteur h₁ définie précédemment. Par exemple, la différence Δh entre h₂ et h₁ peut être comprise entre 1 cm et 3 m, préférentiellement entre 5 cm et 1 m et préférentiellement entre 10 cm et 50 cm.

Ainsi, en imposant une pression supérieure à la pression atmosphérique dans la phase gazeuse du réservoir 9, on peut injecter la sueur 5 du réservoir 9 dans un canal de liaison 10 puis dans un canal principal 14 du dispositif fluidique 4, jusqu'à un orifice 11. A la hauteur h₂ d'un orifice 11, une particule fluide subit au moins deux forces : une force due à la pression imposée par l'actuateur de pression 6, entrainant une particule de fluide vers l'extérieur du canal principal 14 dans le cas d'une pression imposée supérieure à la pression atmosphérique, et une force hydrostatique, entrainant une particule de fluide vers l'intérieur du canal principal dans le cas d'une valeur de Δh positive. Ces deux forces peuvent être de sens opposé et permettre de réaliser un équilibrage fin de la pression à imposer par l'actuateur de pression 6 de manière à maintenir le ménisque à la hauteur d'un orifice 11 en réalisant une boucle d'asservissement fermée. Théoriquement, la pression d'équilibrage ΔPₛₜₒₚ peut être exprimée sous la forme ΔP*ₛₜₒₚ* = *ρ.g*.Δ*h* où ρ correspond à la masse volumique de la sueur 5 et g correspond à la valeur absolue de l'accélération de la pesanteur.

De manière à réaliser une boucle d'asservissement fermée, le système 1 comporte au moins un débitmètre 7. Un débitmètre 7 est agencé dans le système 1 de manière à mesurer un débit ou un débit équivalent au débit d'un canal principal 14. Par exemple, un débitmètre 7 peut être agencé de manière à mesurer le débit d'un canal de liaison 10 ou apte à mesurer le débit dans un canal de liaison 10. Le débitmètre 7 peut déterminer les sens de l'écoulement en transmettant une valeur positive ou négative du débit mesuré et permet préférentiellement de mesurer le débit dans une gamme comprise entre -60 µL/min et 60 µL/min.

Un système 1 comporte également un régulateur par asservissement en boucle fermée. Un régulateur 8 peut par exemple être implémenté par un ordinateur ou un circuit électronique adapté. Un débitmètre 7 est apte à transmettre une information ou une mesure du débit d'un canal principal 4 ou du canal de liaison 10 au régulateur 8. Un régulateur 8 est apte à contrôler, ou transmettre une information de régulation à l'actuateur de pression 6. L'utilisateur peut donner une consigne, par exemple une consigne de débit, au régulateur 8. Une consigne de débit nul peut par exemple être donnée pour maintenir de manière précise un ménisque à la hauteur h₂ d'un orifice 11. Une régulation du débit d'un canal principal 4 en boucle fermée est réalisée par l'action de la force de pression imposée à la sueur 5 et à la rétroaction de la force hydrostatique imposée à la sueur 5. Avantageusement, un régulateur 8 du système 1 est un régulateur dit P.I.D. (acronyme de Proportionnel, Intégral et Dérivée). Une boucle d'asservissement à un débit nul d'un canal principal 4 est peut par exemple être réalisée de la manière suivante : un débitmètre 7 transmet une information du débit au temps t à un régulateur 8. Si le débit est positif, le régulateur 8 régule l'actuateur de pression 6 de manière à diminuer la pression imposée. Si le débit mesuré est négatif, le régulateur 8 régule l'actuateur de pression 6 de manière à augmenter la pression imposée. Cette opération peut être répétée de manière automatique un grand nombre de fois (par exemple plus de dix fois) de manière à converger vers un débit nul ou suffisamment proche de zéro. Par suffisamment proche de zéro, on entend que la valeur absolue du débit moyen, mesuré par exemple comme une moyenne glissante sur une seconde, est inférieure ou égale à 0,200 µL/min, préférentiellement inférieure ou égale à 0,1 µL/min et préférentiellement inférieure ou égale à 0,05 µL/min.

De manière générale, le système permet de paralléliser un contrôle de débit par asservissement d'une pluralité de canaux principaux 4. Un dispositif fluidique 3 comprend avantageusement une pluralité de canaux principaux 4 indépendants, c'est-à-dire non reliés entre eux, chacun des canaux comportant une entrée 14 et un orifice 11. Les différentes sorties d'un actuateur de pression 6 peuvent contrôler indépendamment les pressions imposées dans une pluralité de réservoirs 9 différents. Chaque réservoir 9 peut être relié à une entrée 14 différente par un canal de liaison 10 différent. Une pluralité de débitmètres peuvent chacun mesurer indépendamment le débit d'un canal de liaison 10 différent et transmettre une information de débit à un régulateur 8 comprenant plusieurs entrées. Le régulateur 8 peut comprendre plusieurs sorties reliées à au moins un actuateur de pression 6. De cette manière, il est possible de multiplexer l'évaluation de l'efficacité d'un produit déodorant 2 en utilisant plusieurs orifices 11. Il est par exemple possible d'évaluer l'effet de différents types de sueurs 5 au regard d'un même produit déodorant 2 et/ou l'effet de plusieurs produits déodorant 2 au regard d'un seul type de sueur 5 et/ou de réaliser une variation du type de sueur 5 et du type de produit déodorant 2 au cours d'une même expérience.

Le système peut également comprendre une enceinte 13 à hygrométrie et/ou température commandée, dans laquelle un dispositif fluidique 3 est agencé. L'évaporation, contrôlée en partie par les conditions imposées en température et en hygrométrie, est, à cette échelle, un facteur apte à faire varier de manière significative la position du ménisque de sueur 5 lors de son injection et de sa stabilisation à la hauteur d'un orifice 11. L'enceinte 13 à hygrométrie et/ou température contrôlées permet de se placer dans des conditions expérimentales dans lesquelles il est possible de limiter ou du moins de contrôler cette évaporation.

Le système peut également comprendre un dispositif d'imagerie microscopique 24, apte à imager un ou plusieurs orifices 11 pendant ou après les évaluations de produit déodorant 2. Un dispositif fluidique 3 peut être transparent : dans ce cas, le dispositif d'imagerie microscopique peut également permettre d'observer un canal principal 4 pendant une évaluation d'un produit déodorant 2.

La figure 2 illustre schématiquement une section d'un dispositif fluidique 3. Un dispositif fluidique 13 selon un mode de réalisation de l'invention comporte au moins un canal principal 4 apte à la circulation de sueur 5. La figure 2 illustre une section d'un dispositif fluidique 3 comportant quatre canaux principaux 4. La section est choisie de manière à illustrer la géométrie de chacun des canaux principaux 4. Chaque canal principal 4 comporte une entrée 14 à l'une de ses extrémités et présente un orifice 11 formé par ses parois à une autre de ses extrémités, à une hauteur h₂. Dans une variante, la hauteur de chacun des orifices 11 peut être sensiblement différente de celle des autres orifices 11, mais toujours différente de la hauteur h₁.

Dans un mode de réalisation de l'invention, le dispositif fluidique 3 peut comprendre une première partie 16 monolitique comprenant chaque dit canal principal 4. La première partie 16 peut comprendre une face extérieure 15 sensiblement plane, chaque orifice 11 coïncidant avec ladite face extérieure 15. Cette face extérieure 15 peut permettre l'application d'un produit déodorant 2 sous d'autres formes galéniques qu'une forme liquide. Elle peut être vue comme une modélisation expérimentale de la peau pour l'évaluation d'un produit déodorant 2. Par sensiblement plane, on entend qu'elle peut être comprise localement dans un plan principal ; elle peut être lisse, ou structurée et/ou texturée de manière à mimer les reliefs de la peau. Différentes formes galéniques peuvent être choisies avantageusement parmi un gel, une crème, une émulsion, une mousse et une solution anhydre. Cette caractéristique permet de résoudre le problème de l'art antérieur consistant à ne pas pouvoir évaluer d'autres formes galéniques d'un produit déodorant 2 que la forme liquide. La figure 2 illustre par exemple une couche de produit déodorant 2 déposée sur une face extérieure 15. Cette couche 2 recouvre au moins un orifice 11, et particulièrement l'ensemble des orifices 11 dans l'illustration de la figure 2.

Un capteur peut être avantageusement agencé sur une surface de la première partie 16, en particulier sur une paroi d'un canal principal 4 et/ou une face extérieure 15. Un capteur peut permettre de déterminer un paramètre physico-chimique ou biologique avant, après ou lors d'une réaction entre une sueur 5 et un produit déodorant 2. Avantageusement, un capteur peut être apte à mesurer un pH, une présence ou un type d'acides aminés, une présence ou un type de nucléotides, une enzyme et/ou une bactérie ou une population de bactéries. Sans introduire de limitation sur le type de capteur utilisé, un capteur peut être capacitif, électrochimique, résistif et être implémenté dans un dispositif fluidique 3 par exemple par la microfabrication d'une électrode métallique. Un capteur peut être particulièrement utile pour caractériser les propriétés antibactériennes d'un produit déodorants 2 dépourvu d'action anti-transpirante, et ne conduisant pas à la formation d'un bouchon 25 et/ou de particules formées par floculation.

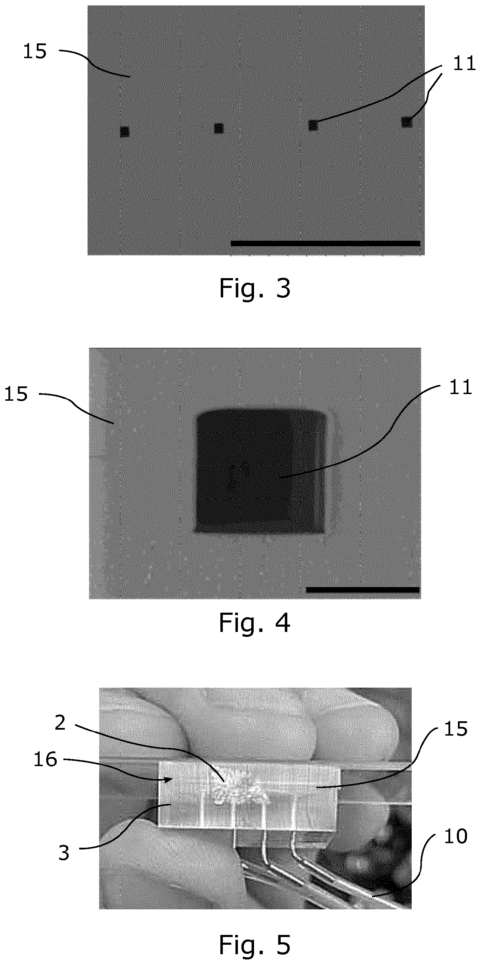

La figure 3 est une photographie réalisée par un microscope électronique à balayage illustrant des orifices 11 d'un dispositif fluidique 3. Plus particulièrement, la figure 3 illustre quatre orifices 11, coïncidant avec une face extérieure 15 plane. La barre d'échelle de la photographie, en bas à droite, correspond à une longueur de 1 mm. Avantageusement, la largeur maximale d'un orifice 11 est inférieure à 1 mm. Elle est préférentiellement comprise entre 1 µm et 1 mm et préférentiellement comprise entre 5 µm et 200 µm. La figure 3 illustre des orifices 11 de forme carré, dont chaque côté mesure sensiblement 60 µm et dont la largeur maximale est sensiblement de 85 µm. Des orifices 11 peuvent être agencés en réseau linéaire, comme illustré dans la figure 3, ou bien en matrice. Ces configurations peuvent permettre d'augmenter la densité d'expériences de bouchage réalisées par unité de surface, par exemple en utilisant un même dépôt de produit déodorant 2 et ainsi obtenir une information statistiquement plus fiable sur des paramètres à mesurer lors des expériences d'évaluation d'un produit déodorant 2.

La figure 4 est une photographie réalisée par un microscope électronique à balayage illustrant un orifice 11 d'un dispositif fluidique 3. Cette photographie est un grossissement de la photographie illustrée en figure 3. La barre d'échelle de la photographie, en bas à droite, correspond à une longueur de 50 µm. La première partie 16 du dispositif fluidique 3 peut être monolithique, c'est-à-dire réalisée en un bloc et/ou en un matériau continu. La première partie 16 peut être réalisée en matériau polymère et préférentiellement élastomère. La figure 3 et la figure 4 illustrent une première partie 16 réalisée en PDMS (acronyme de polydiméthylsiloxane). L'emploi d'un polymère peut permettre de réaliser des orifices 11 coïncidant avec une face extérieure 15 localement plane. Des parois d'un canal principal 4 peuvent par exemple être réalisées à partir d'un moule par polymérisation d'une première couche de PDMS. Une seconde couche de PDMS, plane, peut être assemblée à la première, par exemple par une méthode de cuisson partielle ou par un traitement de surface d'au moins une des surfaces des deux couches par un plasma à oxygène, pour former la dernière paroi d'un canal principale 4. Enfin, le bloc ainsi obtenu peut être coupé dans un plan correspondant à une section normale d'un canal principal 4 à la hauteur d'un orifice 11, de manière à former les orifices 11 et la face extérieure 15 plane. La première partie 16 peut être aussi réalisée en polymère rigide, par exemple en copolymère d'alcène cyclique (ou en anglais *cyclic olefin copolymer,* d'acronyme COC). Dans une variante, la première partie 16 peut être réalisée en métal.

La figure 5 est une photographie illustrant un dispositif fluidique 3 et des canaux de liaison 10. Dans cette photographie, une première partie 16 comporte quatre canaux principaux 4 reliés à quatre canaux de liaison 10 (dont un n'est pas visible sur la figure 5). La figure 5 illustre une surface plane 15 recouverte d'une couche de produit déodorant 2 au moins sur un orifice (non visible à cette échelle).

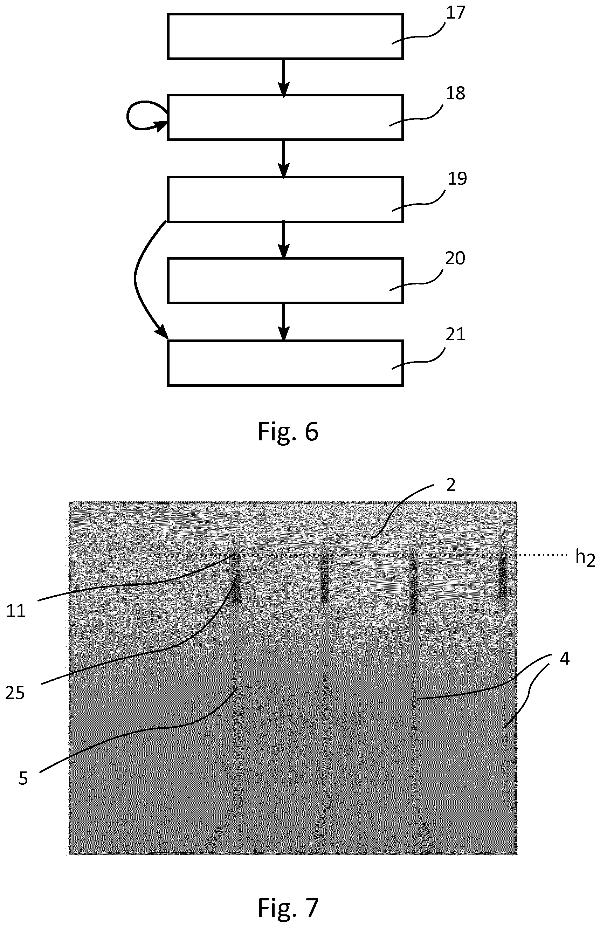

La figure 6 est un schéma illustrant un procédé selon un mode de réalisation de l'invention. Un procédé d'évaluation d'un produit déodorant 2 selon un mode de réalisation de l'invention comporte au moins les étapes suivantes :
- une première étape 17, consistant à introduire (ou à injecter) de la sueur 5 naturelle ou artificielle dans l'ensemble d'un canal principal 4, en imposant une pression dans la phase gazeuse d'un réservoir 9 comportant la sueur 5, avec un actuateur de pression 6, le réservoir 9 étant connecté au canal principal 4. Par « ensemble », on entend que la sueur 5 est injectée depuis l'entrée d'un canal principal 4 jusqu'à son orifice 11 ou plus loin. La sueur peut par exemple déborder d'un canal principal 4 depuis l'orifice 11 ;
- une seconde étape 18, consistant à mesurer un débit de sueur 5 dudit canal principale 4, transmettre l'information dudit débit à un régulateur 8, ledit régulateur imposant une consigne de pression audit actuateur de pression 6 de manière à imposer un débit sensiblement nul au canal principal 4 par asservissement en boucle fermée. Cette étape peut être considérée comme une étape de stabilisation du débit à un débit nul. Par exemple, si le débit est strictement positif, la boucle d'asservissement fermée permet de réduire la pression imposée par l'actuateur de pression 6. Cette boucle peut être répétée un grand nombre de fois, préférentiellement supérieur à 10, avant de mesurer un débit se rapprochant suffisamment de zéro, c'est-à-dire dont la valeur absolue du débit moyen, mesuré par exemple comme une moyenne glissante sur une seconde, est inférieure ou égale à 0,200 µL/min, préférentiellement inférieure ou égale à 0,1 µL/min et préférentiellement inférieure ou égale à 0,05 µL/min. Une fois l'écoulement stabilisé, la pression imposée par l'actuateur de pression 6 peut être maintenue constante et/ou réajustée au cours du procédé en réitérant cette étape;
- une troisième étape 19, consistant à mettre en contact la sueur 5 et un produit déodorant 2 de part et d'autre du ou des orifices 11. De manière générale, on peut déposer un produit déodorant 2 sur les orifices 11. La sueur 5 et le produit déodorant 2 étant mis en contact, une réaction peut être initiée entre les deux.
- une quatrième étape 20, consistant à attendre pendant le déroulement d'une réaction entre ladite sueur 5 et ledit produit déodorant 2. Cette étape peut plus particulièrement consister à attendre pendant la formation d'un bouchon 25 produit par une réaction entre ladite sueur 5 et ledit produit déodorant 2, qui est dans ce cas du type anti-transpirant. Le produit déodorant 2 peut ne pas être assez efficace pour permettre la formation d'un bouchon 25 : cette étape consiste alors seulement à attendre pendant un temps déterminé par l'utilisateur, suffisant pour conclure quant à l'efficacité d'un produit déodorant 2 ;
- une cinquième étape 21, consistant à déterminer un paramètre choisi parmi un paramètre physico-chimique et un paramètre biologique caractéristique d'une réaction entre une sueur 5 et un dit produit déodorant 2. Dans le cas d'un produit anti-transpirant, cette étape peut consister à déterminer au moins un paramètre physico-chimique caractéristique du bouchon 25 et/ou de la réaction entre la sueur 5 et le produit déodorant 2. Dans le cas d'un produit déodorant 2 à effet antibactérien, cette étape peut consister à mesurer l'absence ou la présence d'une population bactérienne dans une sueur 5, un produit déodorant 2 et/ou un produit de réaction, ou de quantifier cette population bactérienne.

De manière générale, les étapes peuvent être mises en œuvre de manière consécutive. En variante, la cinquième étape 21 peut être réalisée en parallèle de la quatrième étape 20. Cette variante peut correspondre avantageusement à une étape pendant laquelle on observe la formation d'un bouchon 25 par imagerie, par exemple au moyen d'un dispositif d'imagerie microscopique 24. En variante, la cinquième étape peut être réalisée en parallèle de l'ensemble des autres étapes du procédé selon un mode de réalisation de l'invention.

La cinquième étape 21 d'un procédé selon un mode de réalisation de l'invention peut avantageusement comprendre une mesure de pH d'une sueur 5 et/ou d'un produit déodorant 2 et/ou du produit d'une réaction entre les deux, avant, au cours et/ou après cette réaction. Cette mesure peut permettre de contrôler que le pH de la sueur 5, d'un produit déodorant 2 et/ou d'un produit d'une réaction entre les deux est compris dans une gamme de pH compatible avec la peau. Cette mesure peut être réalisée par un capteur agencé dans un dispositif fluidique 3 comme décrit précédemment, ou par d'autres moyens, comme une mesure de longueur d'onde émise par un indicateur de pH colorimétrique introduit dans une sueur 5 et/ou un produit déodorant 2.

La cinquième étape 21 d'un procédé selon un mode de réalisation de l'invention peut avantageusement comprendre une mesure de la transmission, la réflexion, la diffusion et/ou de l'absorption, par exemple à une longueur d'onde spécifique, d'une sueur 5 et/ou d'un produit déodorant 2 et/ou du produit d'une réaction entre les deux. Cette mesure peut être réalisée en utilisant un dispositif d'imagerie microscopique 24 et un dispositif fluidique 3 sensiblement transparent, par exemple réalisé en PDMS. Dans le cas d'une présence de particules colloïdales en suspension dans une sueur 5 et/ou d'un produit déodorant 2, cette étape peut correspondre à une mesure de la turbidité de l'une des phases contenue par une première partie 16 ou en surface d'une première partie 16. Cette mesure peut permettre de détecter la présence de particules produites par une réaction entre une sueur 5 et un produit déodorant 2.

La cinquième étape 21 peut également comprendre une mesure de la présence, le type et/ou le nombre de bactéries dans une sueur 5, un déodorant 2 et/ou un produit d'une réaction entre les deux. De cette manière, on peut accéder à des informations sur l'efficacité d'un produit déodorant à action antibactérienne en mesurant le développement d'une population bactérienne dans un dispositif fluidique 3 mimant la peau.

Avantageusement, la sueur 5 en contact avec un orifice 11 à l'extérieur d'un canal principal 4 peut être absorbée, par exemple entre la seconde étape 18 et la troisième étape 19. L'absorption peut être réalisée par l'utilisateur. Elle peut être réalisée par exemple pendant un temps contrôlé et répété entre les expériences. Compte tenu du faible volume sortant d'un canal principal 4 lors de la première étape 17 d'injection, on peut également attendre que la sueur 5 à l'extérieur d'un canal principal 4 s'évapore.

Avantageusement, la troisième étape 19 peut être réalisée en déposant un produit déodorant 2 sur la face extérieure sensiblement plane 15. Le dépôt d'un produit déodorant 2 sur la peau est mimé de cette manière. Lors de cette étape, la face 15 peut être comprise dans un plan horizontal : de cette manière, l'ensemble des orifices 11 peut être à la même hauteur. La face 15 peut aussi être orientée différemment, par exemple être comprise dans un plan vertical.

La figure 7 est une photographie microscopique illustrant la formation de bouchons 25 dans un dispositif fluidique 3. Dans ce mode de réalisation de l'invention, le dispositif fluidique 3 est transparent, en l'occurrence réalisé en PDMS. La ligne en pointillés noir correspond à la face extérieure 15 coïncidant avec les orifices 11, à une hauteur h₂. La photographie de la figure 7 est réalisée après la mise en contact de la sueur 5 avec une couche de produit déodorant 2. Les parties les plus foncées des canaux principaux 4 correspondent aux bouchons 25 formés par une réaction entre la sueur 5 et un produit déodorant 2. Les parties plus claires des canaux principaux 4 correspondent à la sueur 5 liquide. La cinétique de formation des bouchons 25 peut être mesurée en filmant cette partie du système 1 pendant la quatrième étape 20 d'attente.

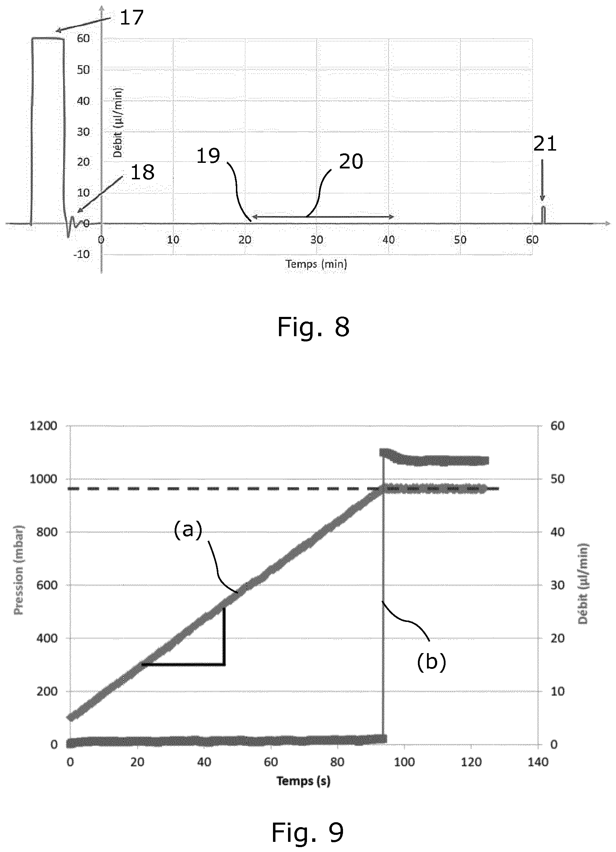

La figure 8 est un diagramme illustrant une mesure du débit dans un canal principal 4 en fonction du temps lors de la mise en œuvre d'un procédé selon un mode de réalisation de l'invention. Le pic de débit formant un créneau pour des valeurs négatives du temps correspond à une injection ou une introduction de sueur 5 dans un canal de liaison 10 et dans un canal principal 4, c'est-à-dire à une première étape 17 du procédé. Lors de la deuxième étape 18 du procédé, la stabilisation du débit d'un canal principal 4 à une valeur sensiblement nulle par un asservissement en boucle fermée correspond aux oscillations de la mesure du débit. Une troisième étape 19 de mise en contact d'un produit déodorant 2 et de sueur 5, à un temps sensiblement égal à 20 minutes, n'est pas visible en mesurant le débit, signifiant que la stabilisation du débit est efficace. La double flèche correspond au temps d'attente de la quatrième étape 20, pendant lequel le produit déodorant 2 est mis en contact avec la sueur 5. De manière générale, la durée de l'attente dans la quatrième étape du procédé peut être supérieure à 10 minutes, préférentiellement supérieure à 20 minutes et préférentiellement supérieure à 1 heure. Elle peut être également égale à plusieurs jours, par exemple égale à deux jours. Le pic de débit observable à un temps supérieur à 60 minutes correspond à une cinquième étape 21, consistant à mesurer un paramètre physico-chimique caractéristique d'un bouchon formé, en l'occurrence la pression de débouchage du canal principal 4.

La figure 9 est un diagramme illustrant une mesure de la pression de débouchage. Cette mesure peut correspondre à une cinquième étape 21 et être réalisée après une quatrième étape 20 d'attente. La courbe (a) de la figure 9 illustre une mesure de la pression (de la phase gazeuse du réservoir 9) imposée par un actuateur de pression 6 au cours du temps dans un canal principal 4 comportant un bouchon 25. L'ordonnée de la courbe (a) est illustrée à gauche du diagramme. La courbe (b) de la figure 9 illustre une mesure du débit du même canal principal 4 au cours du temps. L'ordonnée de la courbe (b) est illustrée à droite du diagramme. La pression imposée par l'actuateur de pression 6 est augmentée dans une première phase. Dans la mise en œuvre de la cinquième étape du procédé, illustrée en figure 9, la pression est augmentée de manière linéaire. Elle peut aussi être augmentée de manière quadratique, exponentielle, logarithmique ou par créneau. Le débit est mesuré simultanément dans le même canal principal 4. Pendant la phase d'augmentation de la pression, le débit reste sensiblement nul en raison de la résistance formée par le bouchon 25. Le début d'une seconde phase correspond au débouchage du canal principal 4, marqué par une variation sensible, préférentiellement une augmentation dramatique du débit mesuré. Le débouchage du canal principal 4 correspond au moment auquel le débit du canal principal dépasse une valeur seuil. Cette valeur seuil peut être égale à 45 µL/min. Elle dépend de la géométrie d'un canal principal 4, de la sueur 5 et du produit déodorant 2 utilisé. L'échantillonnage de la mesure du débit du canal principal peut être réalisé à une fréquence de 10 Hz.

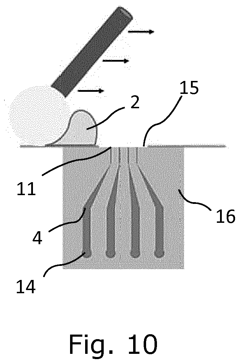

La figure 10 illustre une méthode de dépôt et de mise en contact d'un produit déodorant 2 avec la sueur 5. Les formes galéniques usuelles de produits déodorant 2 peuvent être déposées sur une partie de la face extérieure 15 coïncidant avec des orifices 11. Des formes galéniques de type *RollOn* (dépôt à bille) et de type *Soft Solid Emulsion* (émulsion dont les propriétés mécaniques sont modifiées par des surfactants ou des cires) peuvent être déposées de manière sensiblement uniforme par raclage. Une cale d'épaisseur e₁ (e₁ étant par exemple sensiblement égale à 50 µm) peut être déposée sur la face extérieure 15 d'une première partie 16 d'un dispositif fluidique 3, la cale étant ajourée au dessus du ou des orifices 11. Une cale peut par exemple être réalisée en ruban adhésif. Un produit déodorant 2 peut être raclé sur la cale, le mouvement de raclage étant symbolisé dans la figure 10 par les flèches noires. Une couche sensiblement d'épaisseur e₁ uniforme peut ainsi être déposée sur un ou des orifices 11. L'uniformité du dépôt de produit d'anti-transpirant 2 sur une pluralité d'orifices 11 permet de réaliser des séries d'expériences dans des conditions sensiblement identiques en parallèle.

Dans d'autres modes de réalisation de l'invention, un produit déodorant 2 peut être mis en contact avec la sueur 5 en pulvérisant le produit déodorant 2 sur les orifices 11. Cette méthode permet de mimer la forme galénique du *spray* (pulvérisation) disponible dans le commerce. On peut par exemple déposer une cale ajourée au dessus des orifices 11. On peut disposer le pulvérisateur à une distance apte à déposer un produit déodorant 2 avec une densité sensiblement homogène sur la partie ajourée d'une cale. Préférentiellement, on peut incliner le pulvérisateur, par exemple de 45°, lors de la pulvérisation du produit déodorant 2.

Par ailleurs, les inventeurs remarquent que, sans faire partie des modes de réalisation de l'invention, les caractéristiques du système 1, du dispositif 3 et du procédé selon des modes de réalisation de l'invention peuvent être appliqués à un système d'évaluation du bouchage d'une seringue. En effet, un bouchon peut se former à l'orifice d'une seringue comportant un produit sous forme liquide, par exemple par séchage. Une caractérisation d'un séchage et/ou d'un bouchon peut être réalisée en mesurant la pression de débouchage comme décrit dans la figure 9. De plus, l'imposition d'un débit nul par un système d'asservissement en boucle fermée peut permettre de réaliser des évaluations répétables du séchage et du débouchage d'une seringue. Il suffirait de remplacer le dispositif fluidique 3 par une seringue pour obtenir un système d'évaluation du bouchage d'une seringue. De même, il suffirait de remplacer le dispositif fluidique 3 par une seringue, la sueur 5 par un liquide à injecter et de ne pas mettre en contact le liquide avec un produit déodorant 2 pour obtenir un procédé d'évaluation du bouchage d'une seringue.

## Revendications

1. Système d'évaluation *in vitro* de l'efficacité d'un produit déodorant (2) comprenant :
- au moins un dispositif fluidique (3) comprenant au moins un canal principal (4) comportant une entrée (14) à l'une de ses extrémités et apte à la circulation de sueur (5) naturelle ou artificielle ;
- au moins un réservoir (9) apte à contenir de la sueur (5) naturelle ou artificielle et une phase gazeuse, séparés par une interface (12) à une hauteur h₁,
- au moins un canal de liaison (10) relié à un dit réservoir (9) et à l'entrée (14) d'au moins un dit canal principal (4) ;
- un actuateur de pression (6) apte à imposer la pression d'une phase gazeuse à l'intérieur dudit réservoir (9) ;
**caractérisé en ce qu'**il comporte également :
- au moins un débitmètre (7) ;
- au moins un régulateur (8) par asservissement en boucle fermée ;
un dit débitmètre (7) étant apte à mesurer le débit d'un dit canal principal (4) et à transmettre une information dudit débit audit régulateur (8), ledit régulateur étant apte à transmettre une information de régulation au dit actuateur de pression (6), ledit canal principal (4) présentant un orifice (11) formé par sa paroi à son autre extrémité, à une hauteur h₂, ladite hauteur h₁ étant strictement différente de h₂.

2. Système selon la revendication précédente dans lequel h₂ est strictement supérieure à h₁ et dans lequel un dit actuateur de pression (6) est apte à imposer une pression supérieure à la pression atmosphérique dans un dit réservoir (9).

3. Système selon l'une des revendications précédentes dans lequel ledit dispositif fluidique (3) comporte une pluralité de canaux principaux (4), lesdits canaux principaux (4) étant indépendants.

4. Système selon l'une des revendications précédente dans lequel la largeur maximale d'un dit orifice (11) est inférieure ou égale à 1 mm.

5. Système selon l'une des revendications précédentes dans lequel un dit dispositif fluidique (3) est agencé dans une enceinte (13) dans laquelle au moins un élément choisi parmi l'hygrométrie et la température est commandé.

6. Système selon l'une des revendications précédentes dans lequel un dit dispositif fluidique (3) comprend une première partie (16) monolithique, ladite première partie (16) comprenant chaque dit canal principal (4) et au moins une face extérieure sensiblement plane (15), chaque dit orifice (11) coïncidant avec ladite face extérieure (15).

7. Système selon l'une des revendications précédentes dans lequel ledit dispositif fluidique (3) comporte au moins un capteur choisi parmi un capteur de pH, un capteur d'acides aminés, un capteur de nucléotides, un capteur enzymatique et un capteur de bactéries, chaque dit capteur étant agencé sur une surface choisie parmi une paroi d'un dit canal principal (4) et une dite face extérieure (15).

8. Procédé d'évaluation de l'efficacité d'un produit déodorant mis en œuvre par un système comprenant au moins un dispositif fluidique (13) comprenant au moins un canal principal (4), chaque dit canal principal (4) comprenant une entrée (14) à l'une de ses extrémités et présentant un orifice (11) formé par la paroi dudit canal principal (4) à une autre de ses extrémités à une hauteur h₂, ledit système comprenant au moins un réservoir (9) apte à contenir de la sueur (5) naturelle ou artificielle et une phase gazeuse, séparés par une interface (12) à une hauteur h₁, h₁ étant strictement différente de h₂, comprenant au moins les étapes consistant à :
- introduire de la sueur (5) naturelle ou artificielle dans l'ensemble d'un dit canal principal (4) en imposant une pression dans la phase gazeuse d'un réservoir (9) comportant de ladite sueur (5), avec un actuateur de pression (6), ledit réservoir étant fluidiquement relié audit canal principal (4) ;
- mesurer un débit de sueur (5) dudit canal principal (4), transmettre l'information dudit débit à un régulateur (8), ledit régulateur (8) imposant une consigne de pression audit actuateur de pression, de manière à imposer un débit sensiblement nul audit canal principal (4) par asservissement en boucle fermée ;
- mettre en contact ladite sueur (5) et un produit déodorant (2) de part et d'autre d'au moins un dit orifice (11) ;
- attendre pendant le déroulement d'une réaction entre ladite sueur (5) et ledit produit déodorant (2) ;
- déterminer au moins un paramètre choisi parmi un paramètre physico-chimique et un paramètre biologique caractéristique d'une dite réaction entre ladite sueur (5) et un dit produit déodorant (2).

9. Procédé selon la revendication 8 dans lequel la quatrième étape de la revendication 8 consiste à attendre pendant la formation d'un bouchon (25) produit par une dite réaction.

10. Procédé selon l'une des revendication 8 à 9 dans lequel ledit produit déodorant (2) est sous une forme galénique choisie parmi au moins un gel, une crème, une émulsion, une mousse, une solution anhydre et un spray.

11. Procédé selon l'une des revendications 8 à 10 comprenant une étape consistant à absorber la sueur (5) en contact avec un dit orifice (11) à l'extérieur d'un dit canal principal (4), entre la seconde étape et la troisième étape d'un procédé selon la revendication 8.

12. Procédé selon l'une des revendications 8 à 11 dans lequel la quatrième étape de la revendication 8 consiste au moins à augmenter la pression d'une dite phase gazeuse, mesurer le débit d'un dit canal principale (4) et à mesurer la pression de la dite phase gazeuse lors d'une variation sensible du débit dudit canal principal (4).

13. Procédé selon l'une des revendications 8 à 12 dans lequel on observe la formation d'un dit bouchon par imagerie pendant la quatrième étape de la revendication 8.

14. Procédé selon l'une des revendications 8 à 13 dans lequel la deuxième étape de la revendication 8 est réalisée en pulvérisant un dit produit déodorant (2) sur un dit orifice (11).

15. Procédé selon l'une des revendications 8 à 14 dans lequel un dit dispositif fluidique (3) comprend une première partie (16) monolithique, ladite première partie (16) comprenant chaque dit canal principal (4) et au moins une face extérieure sensiblement plane (15), chaque dit orifice (11) coïncidant avec ladite face extérieure (15) et dans lequel la troisième étape de la revendication 9 est réalisée en déposant un dit produit déodorant (2) sur ladite face extérieure sensiblement plane (15).

16. Procédé selon l'une des revendications 8 à 15 dans lequel la cinquième étape de la revendication 8 comprend une mesure du pH d'au moins un élément choisi parmi une dite sueur (5), un dit produit déodorant (2) et un produit de ladite réaction entre une dite sueur (5) et un dit produit déodorant (5).

17. Procédé selon l'une des revendications 8 à 16 dans lequel ledit dispositif fluidique est sensiblement transparent et dans lequel la cinquième étape de la revendication 8 comprend une mesure d'un élément choisi parmi la transmission et la réflexion d'un éclairage lumineux émis vers au moins un élément choisi parmi une dite sueur (5), un dit produit déodorant (2) et un produit d'une réaction entre une dite sueur (5) et un dit produit déodorant (2).

18. Procédé selon l'une des revendications 8 à 17 dans lequel la cinquième étape de la revendication 8 comprend une détection de la présence de bactéries dans au moins un élément choisi parmi une dite sueur (5), un dit produit déodorant (2) et un produit de ladite réaction entre une dite sueur (5) et un dit produit déodorant (2).

19. Procédé selon la revendication 15 dans lequel la troisième étape de la revendication 9 est réalisée en déposant une cale (22) d'épaisseur e₁ sur ladite face extérieure (15), ladite cale (22) étant ajourée au dessus d'au moins un dit orifice (11), et en raclant un produit déodorant (2) sur ladite cale (22) de manière à déposer une couche de produit déodorant (2) sensiblement d'épaisseur e₁ au dessus d'au moins un dit orifice (11).

## Patentansprüche

1. In-vitro-System zur Beurteilung der Wirksamkeit eines Deodorantprodukts (2), das Folgendes umfasst:
- mindestens eine Fluidvorrichtung (3), die mindestens einen Hauptkanal (4) mit einem Einlass (14) an einem seiner Enden umfasst und dazu in der Lage ist, natürlichen oder künstlichen Schweiß (5) zu zirkulieren;
- mindestens einen Behälter (9), der natürlichen oder künstlichen Schweiß (5) und eine Gasphase enthalten kann, die durch ein Zwischenstück (12) auf einer Höhe h₁ getrennt sind,
- mindestens einen Verbindungskanal (10), der mit dem Behälter (9) und dem Einlass (14) des mindestens einen Hauptkanals (4) verbunden ist;
- einen Druckaktor (6), der den Druck einer Gasphase innerhalb des Behälters (9) ausüben kann;
**dadurch gekennzeichnet, dass** es auch Folgendes umfasst:
- mindestens einen Durchflussmesser (7);
- mindestens eine Steuerung (8) mit geschlossenem Regelkreis;
wobei der Durchflussmesser (7) dazu in der Lage ist, den Durchfluss des Hauptkanals (4) zu messen und Informationen bezüglich des Durchflusses an die Steuerung (8) zu übertragen, wobei die Steuerung dazu in der Lage ist, Steuerungsinformationen an den Druckaktor (6) zu übertragen, wobei der Hauptkanal (4) eine von seiner Wand an seinem anderen Ende gebildete Öffnung (11) auf einer Höhe h₂ aufweist, wobei sich die Höhe h₁ eindeutig von der Höhe h₂ unterscheidet.

2. System nach dem vorhergehenden Anspruch, wobei h₂ eindeutig größer ist als h₁ und wobei der Druckaktor (6) dazu in der Lage ist, einen Druck, der größer als der Atmosphärendruck ist, in dem Behälter (9) auszuüben.

3. System nach einem der vorhergehenden Ansprüche, wobei die Fluidvorrichtung (3) mehrere Hauptkanäle (4) umfasst, wobei die Hauptkanäle (4) unabhängig voneinander sind.

4. System nach einem der vorhergehenden Ansprüche, wobei die maximale Breite der Öffnung (11) kleiner gleich 1 mm ist.

5. System nach einem der vorhergehenden Ansprüche, wobei die Fluidvorrichtung (3) in einem Gehäuse (13) angeordnet ist, in dem mindestens ein Element, ausgewählt aus Feuchtigkeit und Temperatur, gesteuert wird.

6. System nach einem der vorhergehenden Ansprüche, wobei die Fluidvorrichtung (3) einen ersten monolithischen Teil (16) umfasst, wobei der erste Teil (16) jeden Hauptkanal (4) und mindestens eine im Wesentlichen ebene Außenfläche (15) umfasst, wobei jede Öffnung (11) mit der Außenfläche (15) zusammenfällt.

7. System nach einem der vorhergehenden Ansprüche, wobei die Fluidvorrichtung (3) mindestens einen Sensor umfasst, der ausgewählt ist aus einem pH-Sensor, einem Aminosäuresensor, einem Nukleotidsensor, einem Enzymsensor und einem Bakteriensensor, wobei jeder Sensor auf einer Oberfläche angeordnet ist, die ausgewählt ist aus einer Wand des Hauptkanals (4) und der Außenfläche (15).

8. Verfahren zum Bewerten der Effizienz eines Deodorantprodukts, das durch ein System implementiert wird, das mindestens eine Fluidvorrichtung (13) umfasst, die mindestens einen Hauptkanal (4) umfasst, wobei jeder Hauptkanal (4) einen Einlass (14) an einem seiner Enden aufweist und eine von der Wand des Hauptkanals (4) gebildete Öffnung (11) an einem anderen seiner Enden auf einer Höhe h₂ aufweist, wobei das System mindestens einen Behälter (9) umfasst, der dazu in der Lage ist, natürlichen oder künstlichen Schweiß (5) und eine Gasphase zu enthalten, die durch ein Zwischenstück (12) auf einer Höhe h₁ getrennt sind, wobei h₁ sich eindeutig von h₂ unterscheidet, das mindestens die folgenden Schritte umfasst:
- Einführen von natürlichem oder künstlichem Schweiß (5) in den gesamten Hauptkanal (4), indem mit einem Druckaktor (6) ein Druck in der Gasphase eines den Schweiß (5) enthaltenden Behälters (9) ausgeübt wird, wobei der Behälter mit dem Hauptkanal (4) in Fluidverbindung steht;
- Messen eines Durchflusses von Schweiß (5) des Hauptkanals (4), Übertragen von Informationen bezüglich des Durchflusses zu einer Steuerung (8), wobei die Steuerung (8) dem Druckaktor einen Drucksollwert auferlegt, um dem Hauptkanal (4) durch Steuerung mit geschlossenem Regelkreis einen Durchfluss von im Wesentlichen null aufzuerlegen;
- Inkontaktbringen des Schweißes (5) und des Deodorantprodukts (2) auf jeder Seite von mindestens einer Öffnung (11);
- Abwarten des Reaktionsverlaufs zwischen dem Schweiß (5) und dem Deodorantprodukt (2);
- Bestimmen mindestens eines Parameters, ausgewählt aus einem physikalisch-chemischen Parameter und einem biologischen Parameter, der für die Reaktion zwischen dem Schweiß (5) und dem Deodorant (2) charakteristisch ist.

9. Verfahren nach Anspruch 8, wobei der vierte Schritt von Anspruch 8 darin besteht, die Bildung eines durch die Reaktion erzeugten Pfropfens (25) abzuwarten.

10. Verfahren nach einem der Ansprüche 8 bis 9, wobei das Deodorantprodukt (2) in einer galenischen Form vorliegt, die ausgewählt ist aus mindestens einem Gel, einer Creme, einer Emulsion, einem Schaum, einer wasserfreien Lösung und einem Spray.

11. Verfahren nach einem der Ansprüche 8 bis 10, das zwischen dem zweiten und dem dritten Schritt eines Verfahrens nach Anspruch 8 einen Schritt umfasst, der darin besteht, den Schweiß (5), der mit der Öffnung (11) in Kontakt ist, außerhalb des Hauptkanals (4) zu absorbieren.

12. Verfahren nach einem der Ansprüche 8 bis 11, wobei der vierte Schritt von Anspruch 8 zumindest darin besteht, den Druck der Gasphase zu erhöhen, den Durchfluss des Hauptkanals (4) zu messen und den Druck der Gasphase während einer erheblichen Veränderung des Durchflusses des Hauptkanals (4) zu messen.

13. Verfahren nach einem der Ansprüche 8 bis 12, wobei die Bildung des Pfropfens durch Bildgebung während des vierten Schritts von Anspruch 8 beobachtet wird.

14. Verfahren nach einem der Ansprüche 8 bis 13, wobei der zweite Schritt von Anspruch 8 durch Sprühen des Deodorantprodukts (2) auf die Öffnung (11) ausgeführt wird.

15. Verfahren nach einem der Ansprüche 8 bis 14, wobei die Fluidvorrichtung (3) einen ersten monolithischen Teil (16) umfasst, wobei der erste Teil (16) jeden Hauptkanal (4) und mindestens eine im Wesentlichen ebene Außenfläche (15) umfasst, wobei jede Öffnung (11) mit der Außenfläche (15) zusammenfällt und wobei der dritte Schritt von Anspruch 9 durch Abscheiden eines Deodorantprodukts (2) auf der im Wesentlichen ebenen Außenfläche (15) ausgeführt wird.

16. Verfahren nach einem der Ansprüche 8 bis 15, wobei der fünfte Schritt von Anspruch 8 das Messen des pH-Werts von mindestens einem Element umfasst, das ausgewählt ist aus dem Schweiß (5), dem Deodorantprodukt (2) und einem Reaktionsprodukt aus dem Schweiß (5) und dem Deodorantprodukt (2).

17. Verfahren nach einem der Ansprüche 8 bis 16, wobei die Fluidvorrichtung im Wesentlichen transparent ist und wobei der fünfte Schritt von Anspruch 8 das Messen eines Elements umfasst, das ausgewählt ist aus der Übertragung und Reflexion von Lichtbeleuchtung, die in Richtung mindestens eines Elements emittiert wird, das ausgewählt ist aus dem Schweiß (5), dem Deodorantprodukt (2) und einem Reaktionsprodukt aus dem Schweiß (5) und dem Deodorantprodukt (2).

18. Verfahren nach einem der Ansprüche 8 bis 17, wobei der fünfte Schritt von Anspruch 8 das Detektieren des Vorhandenseins von Bakterien in mindestens einem Element umfasst, das ausgewählt ist aus dem Schweiß (5), dem Deodorantprodukt (2) und einem Reaktionsprodukt aus dem Schweiß (5) und dem Deodorantprodukt (2).

19. Verfahren nach Anspruch 15, wobei der dritte Schritt von Anspruch 9 ausgeführt wird, indem ein Plättchen (22) mit einer Dicke e₁ auf der Außenfläche (15) abgeschieden wird, wobei das Plättchen (22) oberhalb mindestens einer Öffnung (11) perforiert ist, und indem Deodorantprodukt (2) auf dem Plättchen (22) abgekratzt wird, um eine Schicht von Deodorantprodukt (2), das im Wesentlichen eine Dicke e₁ aufweist, oberhalb der mindestens einen Öffnung (11) abzuscheiden.

## Claims

1. An *in vitro* system for evaluating the efficacy of a deodorant product (2) comprising:
- at least one fluidic device (3) comprising at least one main channel (4) having an inlet (14) at one end and capable of circulating natural or artificial sweat (5);
- at least one reservoir (9) capable of containing natural or artificial sweat (5) and a gas phase, separated by an interface (12) at a height h₁,
- at least one connecting channel (10) connected to a said reservoir (9) and to the inlet (14) of at least one said main channel (4);
- a pressure actuator (6) capable of imposing the pressure of a gas phase inside said reservoir (9);
**characterized in that** it also comprises:
- at least one flow meter (7);
- at least one closed-loop controller (8);
a said flow meter (7) being capable of measuring the flow rate of a said main channel (4) and transmitting information about said flow rate to said controller (8), said controller being capable of transmitting control information to said pressure actuator (6), said main channel (4) having an orifice (11) formed by its wall at its other end, at a height h₂, said height h₁ being strictly different from h₂.

2. The system according to the preceding claim wherein h₂ is strictly greater than h₁ and wherein a said pressure actuator (6) is capable of imposing a pressure greater than atmospheric pressure in a said reservoir (9).

3. The system according to one of the preceding claims wherein said fluidic device (3) comprises a plurality of main channels (4), said main channels (4) being independent.

4. The system according to one of the preceding claims wherein the maximum width of a said orifice (11) is less than or equal to 1 mm.

5. The system according to one of the preceding claims wherein a said fluidic device (3) is arranged in an enclosure (13) in which at least one element selected from humidity and temperature is controlled.

6. The system according to one of the preceding claims wherein a said fluidic device (3) comprises a first monolithic part (16), said first part (16) comprising each said main channel (4) and at least one substantially flat outer face (15), each said orifice (11) coinciding with said outer face (15).

7. The system according to one of the preceding claims wherein said fluidic device (3) comprises at least one sensor selected from a pH sensor, an amino acid sensor, a nucleotide sensor, an enzyme sensor and a bacteria sensor, each said sensor being arranged on a surface selected from a wall of a said main channel (4) and a said outer face (15).

8. A process for evaluating the efficacy of a deodorant product implemented by a system comprising at least one fluidic device (13) comprising at least one main channel (4), each said main channel (4) having an inlet (14) at one end and having an orifice (11) formed by the wall of said main channel (4) at another end at a height h₂, said system comprising at least one reservoir (9) capable of containing natural or artificial sweat (5) and a gas phase, separated by an interface (12) at a height h₁, h₁ being strictly different from h₂, comprising at least the steps consisting in:
- introducing natural or artificial sweat (5) into the whole of a said main channel (4) by imposing a pressure in the gas phase of a reservoir (9) containing said sweat (5), with a pressure actuator (6), said reservoir being fluidically connected to said main channel (4);
- measuring a sweat (5) flow rate of said main channel (4), transmitting information about said flow rate to a controller (8), said controller (8) imposing a pressure set point on said pressure actuator, so as to impose a substantially zero flow rate on said main channel (4) by closed-loop control;
- bringing said sweat (5) and a deodorant product (2) into contact on either side of at least one said orifice (11);
- waiting during the course of a reaction between said sweat (5) and said deodorant product (2);
- determining at least one parameter selected from a physicochemical parameter and a biological parameter characteristic of a said reaction between said sweat (5) and a said deodorant product (2).

9. The process according to claim 8 wherein the fourth step of claim 8 consists in waiting during the formation of a plug (25) produced by a said reaction.

10. The process according to one of claims 8 to 9 wherein said deodorant product (2) is in a dosage form selected from at least a gel, a cream, an emulsion, a foam, an anhydrous solution and a spray.

11. The process according to one of claims 8 to 10 comprising a step consisting in absorbing the sweat (5) in contact with a said orifice (11) outside a said main channel (4), between the second step and the third step of a process according to claim 8.

12. The process according to one of claims 8 to 11 wherein the fourth step of claim 8 consists at least in increasing the pressure of a said gas phase, measuring the flow rate of a said main channel (4) and measuring the pressure of said gas phase during a substantial variation of the flow rate of said main channel (4).

13. The process according to one of claims 8 to 12 wherein the formation of said plug is observed by imaging during the fourth step of claim 8.

14. The process according to one of claims 8 to 13 wherein the second step of claim 8 is carried out by spraying a said deodorant product (2) onto a said orifice (11).

15. The process according to one of claims 8 to 14 wherein a said fluidic device (3) comprises a first monolithic part (16), said first part (16) comprising each said main channel (4) and at least one substantially flat outer face (15), each said orifice (11) coinciding with said outer face (15) and wherein the third step of claim 9 is carried out by depositing a said deodorant product (2) on said substantially flat outer face (15).

16. The process according to any of claims 8 to 15 wherein the fifth step of claim 8 comprises measuring the pH of at least one element selected from a said sweat (5), a said deodorant product (2) and a product of said reaction between a said sweat (5) and a said deodorant product (5).

17. The process according to one of claims 8 to 16 wherein said fluidic device is substantially transparent and wherein the fifth step of claim 8 comprises measuring an element selected from the transmission and reflection of light illumination emitted towards at least one element selected from a said sweat (5), a said deodorant product (2) and a product of a reaction between a said sweat (5) and a said deodorant product (2).

18. The process according to one of claims 8 to 17 wherein the fifth step of claim 8 comprises detecting the presence of bacteria in at least one element selected from a said sweat (5), a said deodorant product (2) and a product of said reaction between a said sweat (5) and a said deodorant product (2) .

19. The process according to claim 15 wherein the third step of claim 9 is carried out by depositing a shim (22) of thickness t₁ on said outer face (15), said shim (22) being perforated above at least one said orifice (11), and scraping a deodorant product (2) on said shim (22) so as to deposit a layer of deodorant product (2) substantially of thickness t₁ above at least one said orifice (11).
